# EUROPEAN PATENT APPLICATION

(11) **EP 3 187 192 A1**
(43) Date of publication of application: **05.07.2017**
(21) Application number: 15203143.1
(22) Date of filing: 30.12.2015
(51) Int. Cl.: A61K 41/00, A61K 31/12, A61K 31/353, A61K 33/04, A61K 36/185, A61K 36/287, A61K 36/738, A61P 3/10

(54) **PHARMACEUTICAL COMPOSITION FOR USE IN THE TREATMENT OF TYPE II DIABETES MELLITUS**

(71) Applicant: Endocrinology & Metabolism Research Center, Endocrinology & Metabolism Clinical Sciences Institute, 1411413137 Tehran (IR); Tehran University of Medical Science, 1417653955 Tehran (IR)
(72) Inventor: Larijani, Bagher, North Kargar Avenue 1411413137 Tehran (IR); Abdollahi, Mohammad, 1417614411 Tehran (IR); Tabatabaei-Malazy, Ozra, 1411413137 Tehran (IR)
(74) Representative: Scholz, Volker

(57) **Abstract**

The present invention relates to a composition comprising:a)a herbal extract from *Rosa sp., Urticadioica, Tanacetumvulgare* or mixtures thereof, b)curcumin, andc)quercetin as well as to the composition for use in medical treatment, in particular in the treatment of type II diabetes mellitus.

## Description

The present invention relates to a composition, more particular a pharmaceutical composition. In particular, the composition is for use in medical treatment, more specifically in the treatment of type II diabetes mellitus.

### Prior art

Diabetes mellitus (DM) especially type 2 DM is the most common health problem of the world in the current century. Nowadays nearly 387 million people are suffering from DM and 552 million are expected to be affected by diabetes by 2030. Due to the adverse effects of synthetic anti diabetic drugs such as hypoglycemia and lack of the associated drugs safety, considering alternative medicines such as herbal medications for the management of diabetes is necessary. Herbal medications are generally safer, cheaper and are more easily available compared to synthetic drugs. Based on the World Health Organization (WHO) report, 65-80 % of the world's populations are living in developing countries. This population needs to have access to herbal medicines because they have no access to modern medicine. Moreover alternative medicine appears to be more harmonized with patients' beliefs and values. During recent decades, modern medicine has achieved explosive developments, but plants are still a corner stone of healthcare and medical prescriptions. The Evaluation of medicinal plants efficacy for treating disorders such as diabetes has been recommended by WHO. It is established that oxidative stress plays a substantial role in the pathogenesis of diabetes and its complications and antioxidant therapy can protect ß-cells of pancreas against apoptosis and preserve their function. Several epidemiological studies have shown an association between low concentration of antioxidants and the increased risk of diabetes complications. Numerous human and animal studies have demonstrated the positive effects of antioxidant therapy on oxidative stress status, their beneficial influences on ß-cell mass and insulin secretion. Due to the complex pathogenesis of diabetes, multi-models for the management of diabetes will be required. Most of the studies on herbal medicine have suggested antioxidative activity as being the main mechanism. Evaluating the synergistic effects of combined various plants could serve as one of these models.

It is therefore an object of the present invention to provide a (pharmaceutical) composition, in particular a pharmaceutically active mixture, overcoming drawbacks of the prior art, in particular providing strong anti-oxidant properties to be useful in care and management of diabetes. It is a further object to provide a composition helpful to increase insulin secretion in diabetic models.

This object is achieved by a composition comprising: a)a herbal extract from *Rosa sp., Urticadioica, Tanacetumvulgare* or mixtures thereof, b)curcumin, and c)quercetin.

It is preferred that the composition comprises: a)50 to 400 parts by weight of the herbal extract, preferably 100 to 300 parts by weight, more preferably 150 to 250 parts by weight, most preferred about 200 parts by weight, b) 100 to 800 parts by weight of the curcumin, preferably 200 to 600 parts by weight, even more preferred 300 to 500 parts by weight, more preferably 350 to 450 parts by weight, most preferably about 400 parts by weight, and c)10 to 100 parts by weight of the quercetin, preferably 15 to 50 parts by weight, even more preferred 20 to 40 parts by weight, most preferably about 34 parts by weight.

The inventive composition may be prepared by simply mixing the three ingredients a) to c) by any technical means known in the art for preparing respective compositions, such as mixing the components by stirring etc..

Preferably, the herbal extract comprised in the composition is obtainable by a method for preparing a herbal extract, comprising the following steps: a)providing a plant material derived from *Rosa sp., Urticadioica* and/or *Tanacetumvulgare*; b)drying the plant material; c)adding an organic solvent; d)incubating the mixture of plant material and organic solvent; e)obtaining the herbal extract.

More preferred, the plant material derived from *Rosa sp.* is a fruit.

Most preferred, the plant material derived from *Urticadioica* and/or *Tanacetumvulgare* is a leave and/or a small stem.

It is preferred that the drying in step (b) of the method is carried out at a temperature in the range of about 20 to 50°C, preferably of about 37 to 45°C, most preferably of about 42°C.

It is further preferred that the drying in step (b) of the method is carried out for a time period of about 3 to 4 days.

In a preferred embodiment, the organic solvent is ethanol, preferably of about 60 to 96 % (by volume), more preferably of about 80 to 96 % (by volume), most preferably of about 96 % (by volume).

Preferably, the incubating in step (d) of the method is carried out for a time period in the range of about 20 to 40 days, preferably of about 22 to 38 days, most preferably of about 25 to 35 days.

More preferred, the incubating in step (d) of the method is carried out at a temperature in the range of about 20 to 50°C, preferably of about 37 to 45°C, most preferably of about 42°C.

Most preferred, the method additionally comprises the following step: f) adding selenium and/or an organic or inorganic salt thereof.

It is preferred that selenium is added to a concentration of free selenium in the range of about 1 to 100 mg/l, preferably of about 5 to 50 mg/l, most preferably of about 10 to 20 mg/l.

It is further preferred that the method additionally comprises the following step: g)adding urea.

Also preferred, the method additionally comprises the following step:h) exposing the herbal extract to a pulsed electromagnetic field.

More preferred, the electromagnetic field pulse has a sinusoidal, rectangular and/or stochastic shape.

Preferably, the pulsed electromagnetic field has a frequency in the range of about 5 to 750 kHz, preferably of about 50 to 350 kHz, most preferably of about 250 kHz.

It is further preferred that the pulsed electromagnetic field has a power in the range of about 10 to 200 Watt, preferably of about 20 to 100 Watt, most preferably of about 45 Watt.

Most preferred, the pulsed electromagnetic field has a magnetic field strength in the range of 100 to 150 µTesla.

In a most preferred embodiment, the exposing in step (h) is carried out for a time period of about 2 to 5 minutes.

Preferably, the exposing in step (h) is repeated, and preferably is carried out for three times.

The object is further achieved by the inventive composition for use in medical treatment.

Finally, the object is achieved by the inventive composition for use in the treatment of type II diabetes mellitus.

Preferably, the treatment comprises administering to a mammal the composition comprising
a) at least 60 mg of the herbal extract per kg bodyweight of the mammal, preferably at least 100 mg/kg, even more preferred at least 150 mg/kg, most preferred at least about 200 mg/kg,
b) at least 120 mg of curcumin per kg bodyweight of the mammal, preferably at least 150 mg/kg, more preferred at least 200 mg/kg, even more preferred at least 300 mg/kg, most preferred at least about 400 mg/kg; and c) at least 10 mg quercetin per kg bodyweight of the mammal, preferably at least 15 mg/kg, even more preferred at least 20 mg/kg, even more preferred at least 30 mg/kg, even more preferred at least about 34 mg/kg. In the preferred amounts mentioned before, the unit mg/kg refers to the amount of the respective compound in mg related to the bodyweight of the mammal in kg.

Preferably, the inventive composition (or the composition for use) is a pharmaceutical composition. Particularly preferred, the pharmaceutical composition is for oral administration.

The inventors have surprisingly found that the inventive composition solves the problem underlying the present invention by providing a beneficial effect as to insulin secretion in diabetic models and by also providing strong antioxidizing properties which make the inventive composition usefully in the care and management of diabetes.

According to the invention, a pharmaceutical combination of a multi-herbal antioxidant complex (SEQUMIN) is prepared, which included, Setarud (IMOD™), curcumin and quercetin. It was then explored its safety and efficacy in a preclinical trial. The physical interaction of the mixture by spectrophotometer scan showed a synergistic interaction between them. After determination of the maximum and minimum effective doses and pharmacokinetic of the mixture, the acute toxicity (LD50) and biochemical tests were conducted in type 2 diabetic model of rats. The diabetic rats were divided into 5 groups; one group without treatment, one group was treated with glibenclamide, and the others were treated with different dosages of the maximum effective dose of the herbal mixture. The results of blood sugar, LPO (lipid peroxidase), DNA-damage, total antioxidant power, Insulin release, and TNF-a in diabetic rats in comparison with non-diabetics (control group) revealed significant beneficial effects by half-dose of maximum effective dosage of the mixture. The suggested poly-herbal mixture is neither associated with toxicity, nor cell damage. It appeared to be beneficial in hyperglycemia and to reduce oxidative stress damages especially when a half-dose of the maximum effective dosage is used.

The herbal extract from *Rosa sp., Urticadioica* and *Tanacetumvulgare* (herbal extract also being known as IMOD) is described in the application WO 2007/087825 A1, the content of which is incorporated herein by reference.

With respect to the herbal extract and preferred embodiments related thereto, the following definitions are valid.

The term "stochastic shape" comprises the meaning that the electromagnetic field pulse is in the form of a noise. Preferably, the electromagnetic field pulse is of rectangular shape and is combined with a sinusoidal wave inside. The "power" (Watt) of the pulsed electromagnetic field means e.g. effective power. The value of the "magnetic field strength" (Tesla) of the pulsed electromagnetic field indicates e.g. from peak to peak.

The term "pharmaceutical composition", as used herein, is intended to comprise the herbal extract as well as the further constituents including curcumin and quercetin of the present invention.

The pharmaceutical composition can be, for example, in a liquid form, e.g. a solution, syrup, elixir, emulsion and suspension, or in a solid form, e.g. a capsule, caplet, tablet, pill, powder, and suppository. Granules or semi-solid forms and gel caps are also considered. In case that the pharmaceutical composition is a liquid or a powder, the dosage unit optionally is to be measured, e.g. in the dosage unit of a teaspoonful. In addition to the herbal extract or the pharmaceutically active component, the pharmaceutical composition can comprise, for example, flavoring agents, sweeteners, dyes, preservatives, stabilizers, coloring agents, diluents, suspending agents, granulating agents, lubricants, binders and disintegrating agents. A tablet, for example, can be coated. A liquid to be injected should be sterile. Also considered are transdermal delivery systems and liposomal systems. All of the formulations mentioned can be intended for immediate release, timed release and sustained release.

The term "pharmaceutically acceptable", as used herein, means at least non-toxic. The "pharmaceutically acceptable carrier", as meant in the present disclosure, may take a wide variety of forms depending upon the desired route of administration. The term comprises conventional pharmaceutical diluents such as water or ethanol and conventional tableting ingredients such as corn starch, lactose, sucrose, sorbitol, talc, stearic acid, magnesium stearate, dicalcium phosphate or gums.

Administration of the pharmaceutical composition of the present invention can use different routes, such as oral, sublingual, parenteral, intravenous, intraperitoneal, nasal, vaginal, rectal, subcutaneous, intradermal, intramuscular and topic. A dosage unit can be administered once or several times a day, week or month. The delivery can also be continuously by infusion or through a transdermal sustained release system, for example.

The present invention provides a composition comprising a combinatory herbal extract from *Rosa sp., Urticadioica*and/or*Tanacetumvulgare* which may be treated by electromagnetic field radiation together with curcumin and quercetin.

In the following, the invention will be described in greater detail by means of examples and by referring to the figures, wherein
Fig. 1: UV absorbance of Quercetin
Fig. 2: UV absorbance of Curcumin
Fig. 3: UV absorbance of IMOD
Fig. 4: UV absorbance of IMOD + Curcumin
Fig. 5: UV absorbance of IMOD + Quercetin
Fig. 6: UV absorbance of Curcumin + Quercetin
Fig. 7: UV absorbance of IMOD + Curcumin + Quercetin
Fig. 8: MTT assay in untreated or treated with each herbal component an islet cells of healthy rats (A=IMOD, B= Quercetin, C=Curcumin)
Fig. 9a, b: MTT assay with maximum and minimum effective doses (IQ=IMOD + Quercetin, IC= IMOD +Curcumin, QC= Quercetin + Curcumin, Combination= Mixture of all 3 compounds), respectively, in healthy rats
Fig. 10a, b: ROS formation with maximum and minimum effective doses (IQ=IMOD + Quercetin, IC=IMOD + Curcumin, QC= Quercetin + Curcumin, Combination= Mixture of all 3 compounds), respectively in healthy rats
Fig. 11a, b: Insulin release (GSIS) test with maximum and minimum effective doses (IQ=IMOD +Quercetin, IC= IMOD + Curcumin, QC= Quercetin + Curcumin, Combination= Mixture of all 3 compounds), respectively in healthy rats
Fig. 12: The effect of different treatment regimens onbloodglucose of rats.
Fig. 13: The effect of different treatment regimens onDNA-Damage of rats.(Data are presented as mean± SE; a P<0.01 vs. control group; b P<0.001vs. diabetic group;
P<0.001vs.diabetic+Gli group; d P<0.001 vs. diabetic+half dose group).
Fig. 14: The effect of different treatment regimens an TNF-a in rats.(Data are presented as mean± SE; a P<0.001 vs. control group; b P<0.001 vs. diabetic group; P<0.001 vs.diabetic+Gli group; d P<0.001 vs. diabetic+half dose group.)
Fig. 15: The effect of different treatment regimens on Total Antioxidant Power in rats. (Data are presented as mean± SE; a P<0.001vs. control group; b P<0.001vs. diabetic group).
Fig. 16: The effect of different treatment regimens an LPO in rats. (Data are presented as mean± SE; a P<0.01vs. control group; b P<0.001vs. diabetic group).
Fig. 17: The effect of different treatment regimens an Insulin release in rats. (Data are presented as mean± SE; a P<0.001 vs. control group; b P<0.001 vs. diabetic group).

The invention will now be described in more detail by the following examples with the intention to exemplify the invention. The examples, however, are not intended to have any limiting effect on the subject-matter of the claims or on the scope of protection.

### Preparation of raw herbal extracts

Leaves and small stems of nettle (Urticadioica) and tansy (Tanacetumvulgare) are collected from wild fields. After separation of useful parts and initial cleaning, the material is dried on a wooden network in a dark place for 3-4 days, preferably at 42°C. In dried condition, the plant material should be green without any change in colour, and leaves and stems should be brittle. For extraction, airtight glass vessels are used. The dried plant material is broken into small pieces (2-5 cm) and placed into the glass vessels such that there is no space left. After packing (compressing), EtOH (96 %; herein, % of an ethanolic solution refers to % by volume)) is added until the vessel is filled completely. The vessels are placed into an incubator (37-45°C, preferably 42°C) for 20-40 days until a dark green solution appears.

For the extraction of wild rose (Rosa canina), dried fruits are used. In further embodiments, other species of Rosa sp. can be used alternatively or in addition. The fruits are filled into airtight vessels up to a half and EtOH is added. The vessels are kept in an incubator (37-45°C, preferably 42°C) for 20-40 days until an orange-red colored extract appears.

After the incubation period, when the plant material is colourless, the extracts are collected by separating them from plant material using a cloth filter.

### Electromagnetic treatment

The extract of Rosa canina is exposed to an electromagnetic field for 3 min. Then, 50-70 ml of the radiated Rosa canina extract is transferred to 2 1 of Urticadioica and Tanacetumvulgare extracts, respectively. To each liter of the combined Urtica/Rosa and Tanacetum/Rosa extracts, respectively, 16 mg of selenium and 150 mg urea is added. In alternative embodiments, either selenium or urea is added. Then, the vessels are sealed again and kept in the incubator for 24 h at 42°C. After incubation, the vessels are exposed 4 times to an electro-magnetic field, 3 min each, and are pooled together. The resulting extract is passed sequentially through a 5, 0.45 and 0.22 µm filter, respectively, and partitioned to sterile vials. After labelling and packaging, the herbals extract is ready for use.

The electromagnetic field, to which the raw extracts are exposed, is pulsed, powerful and monopolar in that the direction of the electric current generated in a Magnetic Impulse Generator (MIG) apparatus doesn't change. The pulsed magnetic field has a very high frequency ranging from 5 kHz-750 kHz. In this example, the pulse of a rectangular shape used. Nevertheless, in other embodiments, a sinusoidal or stochastic shape is considered as well. Preferably, the pulse is of rectangular shape and is combined with a sinusoidal wave inside. Although it is not intended to be bound to any theory, it is hypothesised that the special kind of the produced pulse causes some changes in the physical configuration of atoms in the molecules and/or arrangements of molecules thus leading to altered chemical properties.

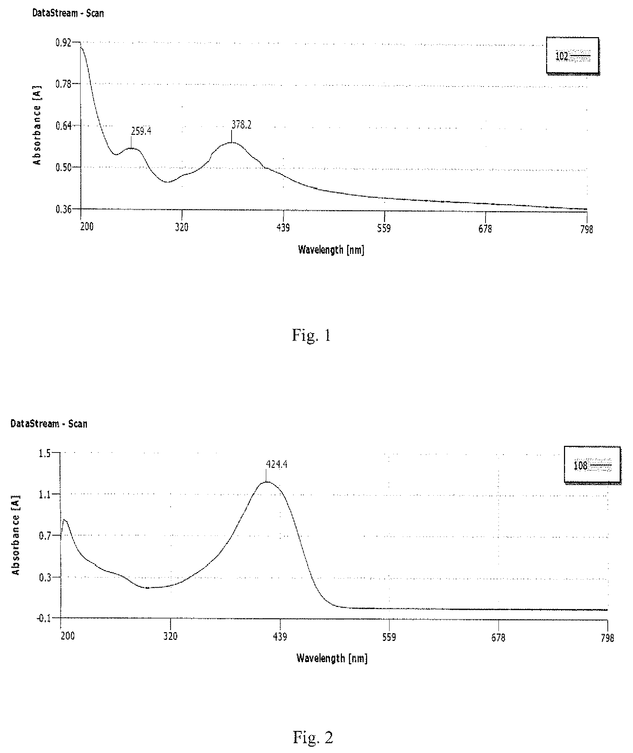

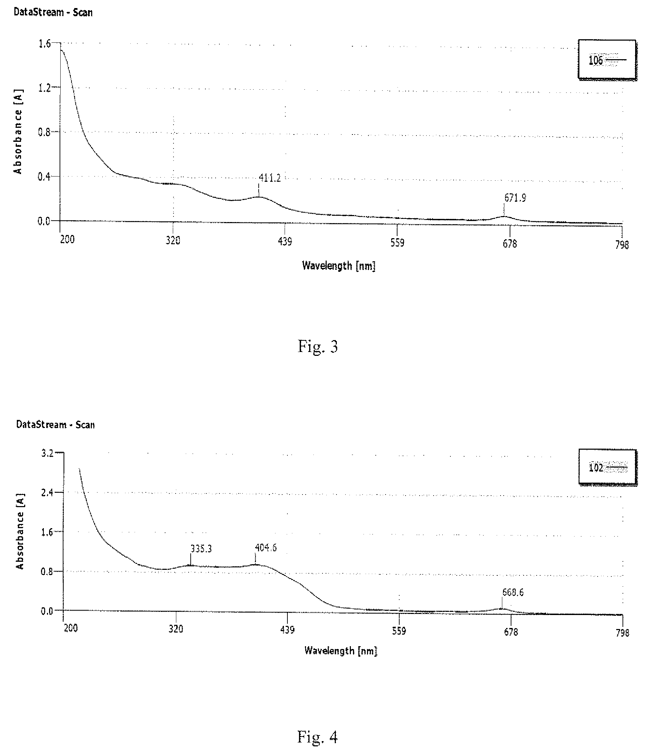

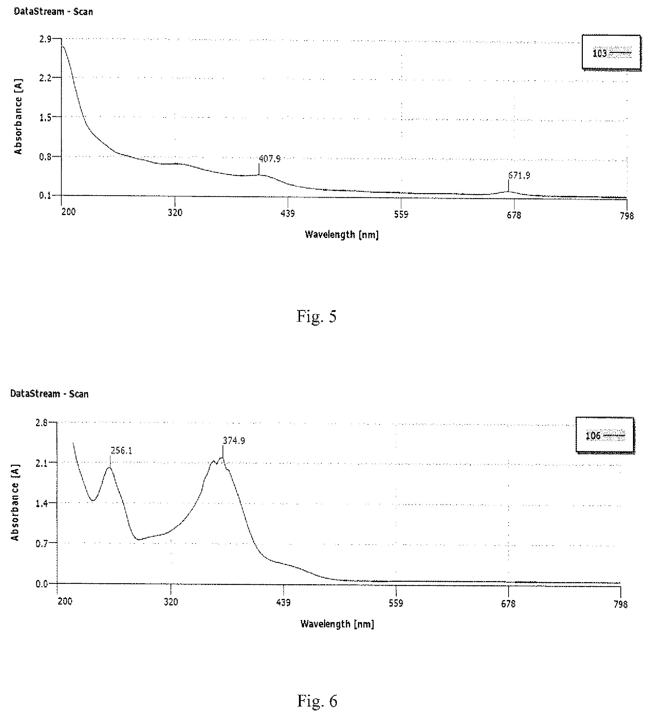

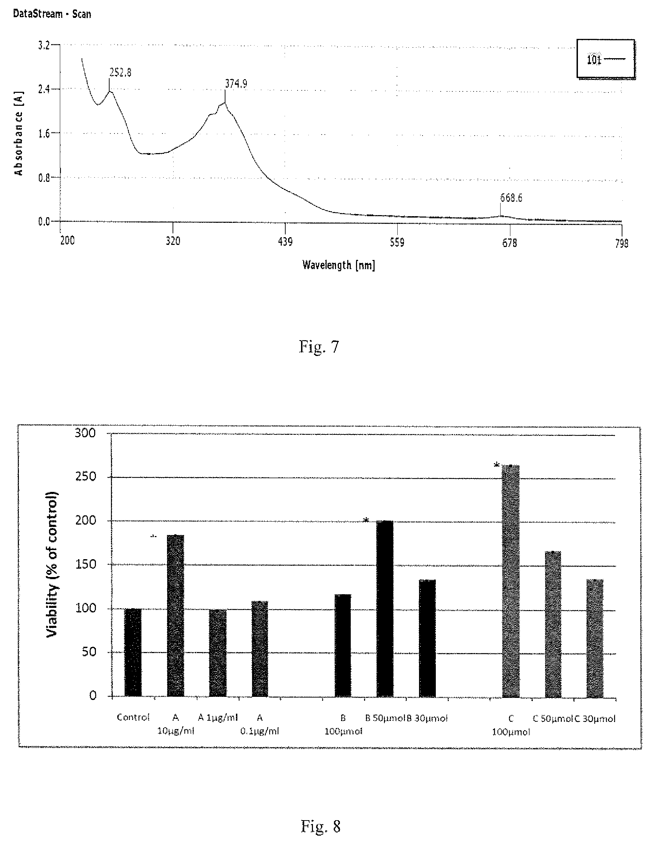

In the preparation of the herbal extracts, 3-4 times radiation of electromagnetic pulses of high frequency is used for 2-5 min each. The electrical power (e.g. effective power) of the pulses is about 20 to 100 Watt, and the best effect is obtained at 45 Watt.
a) A step-by-step approach was designed for preparing a new anti-diabetic drug.
b) A systematic review was primarily conducted for choosing the best herbal combination which has anti-inflammatory and antioxidative properties besides having a hypoglycemic activity.
b) Followed by collecting evidences form literature on safety and efficacy of each component, the proposed mixture was extracted using the systematic review approach in validated international web databases; PubMed, Web of Science, Scopus, and Google Scholar, through which the safety and efficacy of each component is confirmed.
c) Breaded idea extracted systematic electronic search to cell and subsequent animal studies.
d) The plant mixture components, including IMOD, Curcumin, and Quercetin are prepared. IMOD is patented previously by the application number of "VVO 2007087825 A1".
e) Physical interaction of each component of the herbal mixture is assessed by spectrophotometer scan (CECIL, Swiss) and their UV Test was drawn in 200-800 nm (Figures 1-3).
f) Physical interaction of combination of two components of the herbal mixture is assessed by spectrophotometer scan (CECIL, Swiss) and their UV Test drawn was in 200-800 nm (Figures 4-6).
g) The physical interaction of combination of all 3 ingredient of the herbal mixture is assessed by spectrophotometer scan (CECIL, Swiss) and drawing its UV Test in 200-800 nm (Figure 7). Overall, the physical interaction of the mixture assessed by spectrophotometer scan was shown a synergistic interaction between all of its compounds.
h) Conducting an in vitro study by the herbal mixture, the maximum and minimum effective doses of each compound were determined based on collected data from previous in vitro studies through an electronic systematic search.
i) Maximum, minimum, and medium doses of the determined range of the effective dose was compared to assess its efficacy an islet cells of pancreas of healthy rats in 4 groups introduced as control (without treatment) group, or treatment with IMOD, Curcumin, or Quercetin in separated groups. This comparison was performed to assess the viability of islet cells by MTT (Methyl Tetrazolium) test (Figure 8). This test was repeated 3 times.

### Islet Isolation

In accordance with the ethical guidelines in animal research, Rat islets were isolated from male adult Wistar rats (>12 weeks) weighting 200-250 g. The study was also approved by the institute (code: TUMS- EC-00263). Islets were isolated from the pancreas using collagenase digestion followed by repeated dextran gradient centrifugation. Animals were anesthetized with intraperitoneal injection of sodium pentobarbital (60 mg/kg). After laparotomy, the common bile duct was ligated at its leave the liver. The duct was cannulated at it left duodenum and then the pancreas was perfused by the injecting of 10 mL of Krebs buffer. Perfused pancreas was dissected from duodenum and the tissue surrounding the islet was digested by break down using collagenase, and after separation from fat, the tissue was washed by Krebs-N-2-Hydroxyethylpiperazine-N'-2-Ethanesulfonic Acid (HEPES) buffer three times. The extract tissue was centrifuged in Krebs buffer for two rounds of 60s and then 0.5% bovine serum albumin (BSA) was added for the completion of digestion. Finally, the islets were separated from the remaining tissue by hand-picking under stereomicroscope and the purity was evaluated by dithizone staining as being >95%. Those islets between 100 and 150 mm in diameter were used for the purpose of our experiments.

### Islets viability assay (MTT)

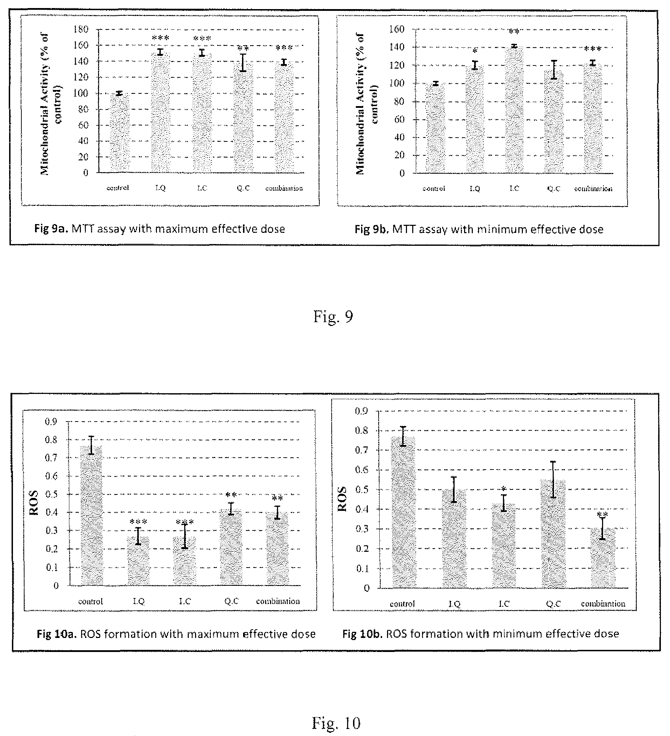

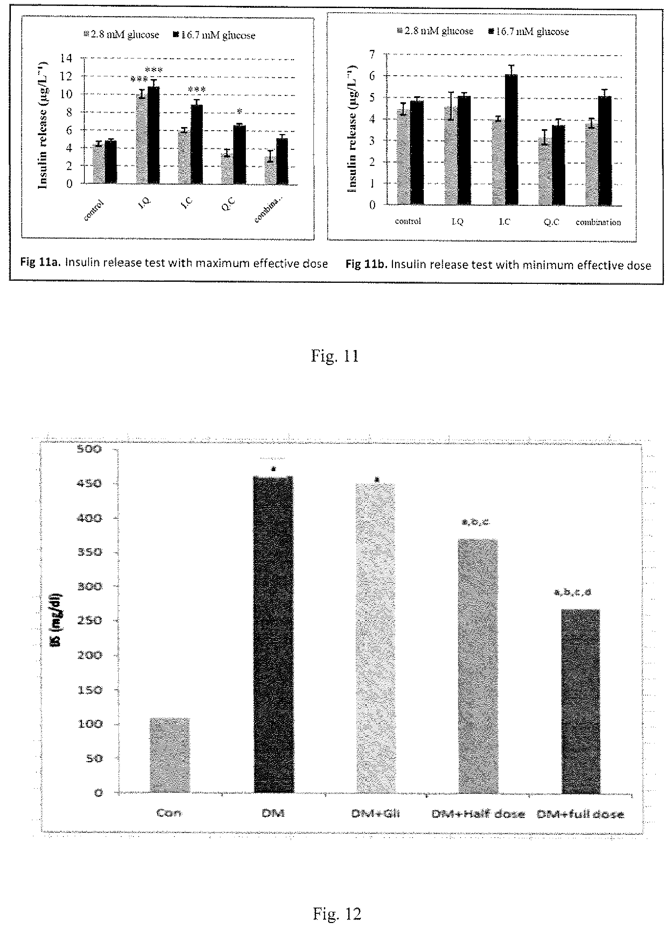

This assay is based on the reduction of 3-4, 5-dimethylthiazol-2-yl-2, 5-diphenyltetrazolium bromide (MTT), a yellow tetrazole to purple formazan by mitochondrial respiration in viable cells. After removing the medium, pretreated islets were washed twice by Krebs-HEPES buffer, and 20 mL of MTT solution was added and incubated for 4 h at 37 °C. After washing, the formazan was resuspended in 100 mL dimethyl sulfoxide and the absorbance was measured at 570 nm by ELISA reader. The viability of the groups was shown as the percentage of controls that is assumed as 100%.
j) Followed by determining the maximum effective dose of each component-induced maximum percent of islet cell viability, it is reasonable to measure MTT, Reactive Oxygen Species (ROS) formations, and insulin release by means of measuring Glucose Stimulated Insulin Secretion (GSIS) using 2.8 and 16.7 mM glucose on the combination of two and finally three components of the herbal mixture in healthy rats (Figures 9-11a, b).

### Measurement of Reactive Oxygen Species (ROS) formation

To measure reactive oxygen species (ROS) generation, a fluorometric assay using intracellular oxidation of 2, 7-dichlorofluoroscein diacetate (DCFH-DA) was performed which was set up previously for this purpose in our laboratory. Pretreated islets were washed with phosphate-buffered saline (PBS), and then were incubated with 40 mM DCFH-DA for 30 min. At the end of incubation, the islets were washed with PBS, lysed with NaOH, and then aliquots were transferred into the blank well plate. Then, the fluorescence of dichlorofluoroscein, which is the oxidized product of DCFH-DA, was measured by ELISA fluorimeter using excitation and emission wave lengths of 485 and 530 nm, respectively. The values were standardized by the amount of total protein per well.

### Glucose-Stimulated Insulin Secretion (GSIS) assay

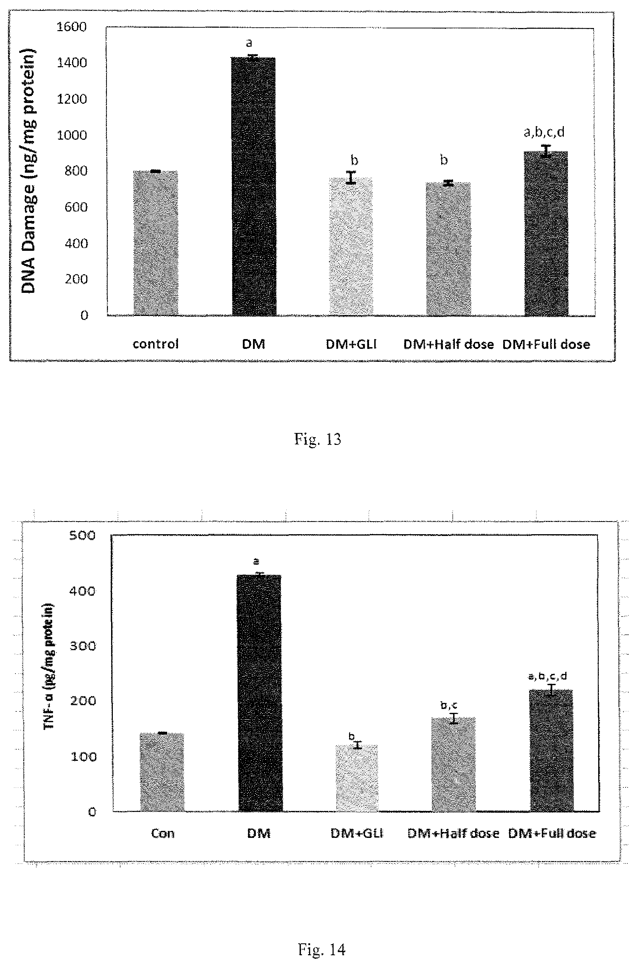

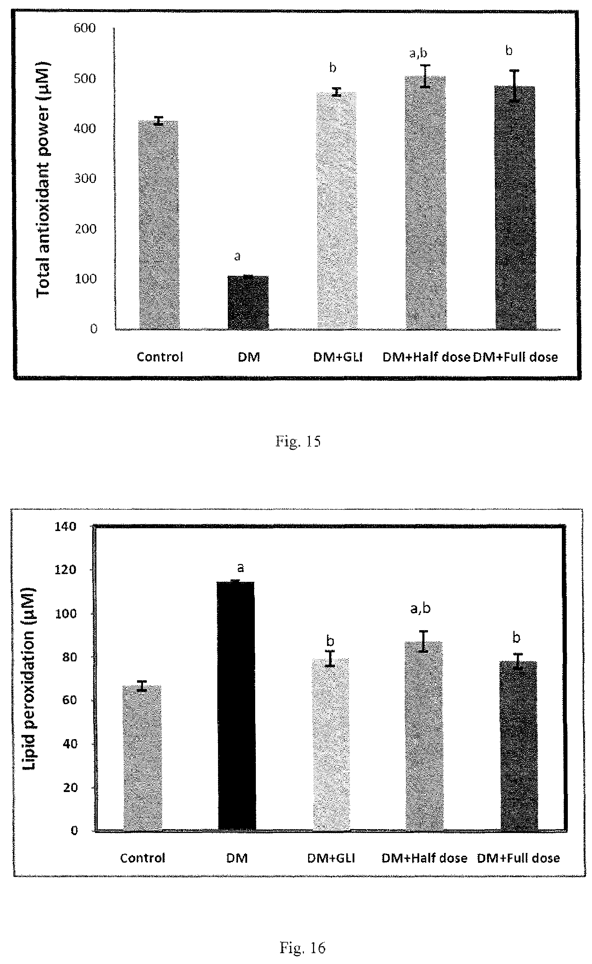

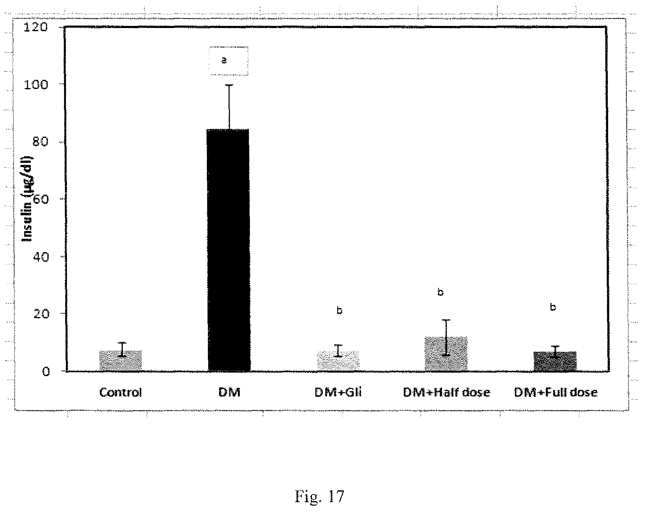

Pretreated islets were separated in tubes and were washed twice by Krebs-HEPES buffer and then were incubated for 30 min at 37 °C at a level of 2.8 mM glucose. An amount of 1 mL of 2.8 mM glucose and 16.7 mM glucose as basal and stimulant doses, respectively, was added to all the groups and was incubated at 37 °C for 30 min. Then, the supernatants were collected and stored in separate microtubes. Insulin concentration was determined by rat insulin ELISA kit according to the manufacturer's protocol (DiaMetra, Italy). Results are displayed as microunits insulin secreted per islet per hour.
k) In accordance with the ethical committee approval, an in vivo two-phase study was conducted in rats. In the first phase, the pharmacokinetic behavior of the mixture in different doses, the safety, toxicity, and also mortality rates were determined. As well, the second phase was carried out to assess biochemical parameters including, blood sugar, lipid peroxidase (LPO), DNA-damage (8-hydroxy-desoxyguanosine/ Elisa/CUSABIO/Japan), Total antioxidant power (FRAP), insulin release (Elisa/Italy), and TNF-a (Sigma/USA). The ethical guidelines are considered throughout the animal study.
k) Aiming to assess the pharmacokinetic behavior of the mixture in the first phase of in vivo study, DM was induced in fasted male adult Wistar rats (>12 weeks) weighting 200-250 g using single intraperitoneal injection of STZ (50 mg/kg) and alloxan (80 mg/kg).
   Within 1 wk of injection, rats showing blood glucose values 220 mg/dL were diagnosed as DM by BIONIME glucometer, and were accordingly included in the study.
   Diabetic animals were randomly divided into 5 groups with 5 rats in each group. In addition, we considered 5 non-DM male adult Wistar rats as healthy control group. Diabetic animal groups were stratified as follows: without treatment, under treatment with glibenclamide (7.5 mg/kg), 1/3 of full dose, 1/2 of full dose, and the total of full dose of the mixture. Data extracted from systematic electronic search of previous in vivo studies conducted in each compound was used to determine the full dose of the herbal mixture. On that account, the full dose was taken as 200 mg/kg IMOD + 34 mg/kg Quercetin + 400 mg/kg Curcumin for oral administration. The above protocol was considered in different times; before treatment (0), and in 1h, 3h, 5h, 7h, 12h, and 24h after treatment for all groups. We didn't find any beneficial effect with herbal mixture at times more than 7h after treatment, nor with 1/3 of full dose (Figure 12). At the end of 24h study, mortality rate was reported as 3 rats by glibenclamide, 3 rats by full dose, 2 rats by 1/3 of full dose, and 2 rats by 1/2 of full dose.
k) b: The second phase of in vivo study was started followed by the reported safety confirmation of the mixture. In this phase another male adult Wistar rats (>12 weeks) weighting 200-250 g was considered to be induced DM by single intraperitoneal injection of STZ (50 mg/kg) and alloxan (80 mg/kg). Within 1 wk of injection, rats showing blood glucose values 220 mg/dL were diagnosed as DM by BIONIME glucometer and were included in the study. Diabetic animals were randomly divided into 4 groups with 5 rats in each group. In addition, we considered 5 non-DM male adult Wistar rats as healthy control group. Diabetic animal groups were divided as follows: without treatment, under treatment with glibenclamide (7.5 mg/kg), 1/2 of full dose or total full dose of the mixture for oral administration. At 7h after treatment, the animals were anesthetized with an intramuscular (im) injection of ketamine (4 mg/100 g) and xylazine (1 mg/100 g) to prepare them for sample collection. Blood was collected from the heart into a heparinized syringe. Blood samples were centrifuged at 1200 g for 10 min at 4°C and plasma was frozen at -80°C until use. Subsequently, LPO, 8-OHdG, FRAP, Insulin release, and TNF-a were measured 3 times for accuracy purposes (Figures 13-17, and Table 1).

### Total antioxidant power (FRAP)

Reduction of Fe3+ to Fe2+ by the biological sample is an indicator of antioxidant capacity. The complex between Fe2+ and TPTZ produces a blue color with a maximum absorbance at 593 nm.

### Lipid peroxidation (LPO)

Thiobarbituric acid-reactive substances (TBARs) were measured using 1, 1', 3, 3'-tetraethoxypropane as a standard and from a standard curve of TBA adduct formation.

**Table 1. Summary of results by half-dose of the maximum effective dosage of the mixture in T2DM rats compared to non-diabetic rats (control group)**

| **Variable** | **P-valueofcomparison** |
|---|---|
| **Blood sugar** | **P<0.01** vs. control group |
| | **P<0.001vs**. diabetic non-treatment group |
| | P<0.001 vs. diabetic+ glibenclamide **diabetics** |
| **LPO** | **P<0.01** vs. control group |
| | P<0.001 vs. diabetic non-treatment group |
| **DNA-damage** | **P<0.001vs. diabetic non-treatment group** |
| **Insulin release** | **P<0.001vs. diabetic non-treatment group** |
| **TNF-a** | **P<0.001 vs. diabetic non-treatment group P<0.001vs. diabetic+ glibenclamide diabetics** |
| **Total antioxidant Power** | **P<0.001vs. control group** |
| | **P<0.001vs. diabetic non-treatment group** |

With respect to the above explanation of new invention, some differences are unfolded which distinct our recent invention with the prior art as below:
1) Focus on the oxidative stress as the main pathophysiology of diabetes and its complications while selecting herbal species for the purpose of present invention, i.e. the inventive composition.
2) Conduction a wide electronic systematic search to find hypoglycemic antioxidative herbal medicines with the beneficial effect on the insulin secretion of islet cells of pancreas.
3) Reviewing the prior studies with stronger evidence-based documents to prevent repeating in vitro and in vivo studies and also saving time.
4) Recruiting those plants with synergistic effects on each other to enhance their combined effects. In addition, choosing plants which having the maximum therapeutic effects and the minimal side effects is considered.
5) Choosing the best herbal species for combination proposes that besides having hypoglycemic activity and antioxidative properties, have anti-inflammatory activity, too.
6) The most innovative point of the present study comes from the rational step-by-step design procedure of a new anti-diabetic drug which includes breeding the idea through systematic electronic search to cell and subsequent animal studies that ultimately leads to different levels of clinical trials. It should be not forgotten that its clinical trials are ongoing. Although many herbal preparations have been proposed for diabetes, none of them have had comprehensive and simultaneous investigations at various cellular-molecular and animal levels.
7) The other significant respect which makes this work innovative is the attention paid to the kinetics and safety of the proposed herbal combination as part of the standard process of drug production and manufacturing.
8) Other strength points of the present study are the process of assessing the anti-inflammatory properties of the herbal combination through measuring TNF-a, and also investigating FRAP as a strong marker for identifying the anti-stress effects of oxidative stress besides the common and prevalent previous markers (LPO).

The features disclosed in the foregoing description, in the claims and the accompanying drawings may, both separately and in any combination be material for realizing the invention in diverse forms thereof.

## Claims

1. Composition comprising:
a) A herbal extract from *Rosa sp., Urticadioica, Tanacetumvulgare* or mixtures thereof,
b) Curcumin, and
c) Quercetin.

2. Composition according to claim 1, comprising:
a) 50 to 400 parts by weight of the herbal extract,
b) 100 to 800 parts by weight of the curcumin, and
c) 10 to 100 parts by weight of the quercetin.

3. Composition according to claim 1 or 2, wherein the herbal extract comprised in the composition is obtainable by a method for preparing a herbal extract, comprising the following steps:
a) providing a plant material derived from *Rosa sp., Urticadioica* and/or *Tanacetumvulgare;*
b) drying the plant material;
c) adding an organic solvent;
d) incubating the mixture of plant material and organic solvent;
e) obtaining the herbal extract.

4. The composition according to any of the claim 3, wherein the organic solvent is ethanol, preferably of about 60 to 96 % (by volume), more preferably of about 80 to 96 % (by volume), most preferably of about 96 % (by volume).

5. The composition according to any of the claims 3 or 4, the method additionally comprising the following step:
f) adding selenium and/or an organic or inorganic salt thereof.

6. The composition according to claim 5, wherein selenium is added to a concentration of free selenium in the range of about 1 to 100 mg/l, preferably of about 5 to 50 mg/l, most preferably of about 10 to 20 mg/l.

7. The composition according to any of the claims 3 to 6, the method additionally comprising the following step:
g) adding urea.

8. The composition according to any of the claims 3 to 7, the methodadditionally comprising the following step:
h) exposing the herbal extract to a pulsed electromagnetic field.

9. The composition according to claim 8, wherein the electromagnetic field pulse has a sinusoidal, rectangular and/or stochastic shape.

10. The composition according to claim 8 or 9, wherein the pulsed electromagnetic field has a frequency in the range of about 5 to 750 kHz, preferably of about 50 to 350 kHz, most preferably of about 250 kHz.

11. The composition according to any of claims 8 to 10, wherein the pulsed electromagnetic field has a power in the range of about 10 to 200 Watt, preferably of about 20 to 100 Watt, most preferably of about 45 Watt.

12. The composition according to any of claims 8 to 11, wherein the pulsed electromagnetic field has a magnetic field strength in the range of 100 to 150 µTesla.

13. Composition according to any of the preceding claims for use in medical treatment.

14. Composition according to any of the claims 1 to 12 for use in the treatment of type II diabetes mellitus.

15. Composition for use in treatment of type II diabetes mellitus according to claim 14, wherein the treatment comprises administering to a mammal,the composition comprising
a) at least 60 mg of the herbal extract per kg bodyweight of the mammal,
b) at least 120 mg of curcumin per kg bodyweight of the mammal; and
c) at least 10 mg quercetin per kg bodyweight of the mammal.

## Amended claims

### Amended claims in accordance with Rule 137(2) EPC.

1. Composition comprising:
a) 150 to 250 parts by weight of a herbal extract from *Rosa sp., Urticadioica*, *Tanacetumvulgare* or mixtures thereof,
b) 350 to 450 parts by weight of Curcumin, and
c) 20 to 40 parts by weight of Quercetin.

2. Composition according to claim 1, wherein the herbal extract comprised in the composition is obtainable by a method for preparing a herbal extract, comprising the fellow-ing steps:
a) providing a plant material derived from *Rosa sp*., *Urticadioica* and/or *Tanacetumvulgare*;
b) drying the plant material;
c) adding an organic solvent;
d) incubating the mixture of plant material and organic solvent;
e) obtaining the herbal extract.

3. The composition according to any of the claim 2, wherein the organic solvent is ethanol, preferably of about 60 to 96 % (by volume), more preferably of about 80 to 96 % (by volume), most preferably of about 96 % (by volume).

4. The composition according to any of the claims 2 or 3, the method additionally comprising the following step:
f) adding selenium and/or an organic or inorganic salt thereof.

5. The composition according to claim 4, wherein selenium is added to a concentration of free selenium in the range of about 1 to 100 mg/l, preferably of about 5 to 50 mg/l, most preferably of about 10 to 20 mg/l.

6. The composition according to any of the claims 2 to 5, the method additionally comprising the following step:
g) adding urea.

7. The composition according to any of the claims 2 to 6, the methodadditionally comprising the following step:
h) exposing the herbal extract to a pulsed electromagnetic field.

8. The composition according to claim 7, wherein the electromagnetic field pulse has a sinusoidal, rectangular and/or stochastic shape.

9. The composition according to claim 7 or 8, wherein the pulsed electromagnetic field has a frequency in the range of about 5 to 750 kHz, preferably of about 50 to 350 kHz, most preferably of about 250 kHz.

10. The composition according to any of claims 7 to 9, wherein the pulsed electromagnetic field has a power in the range of about 10 to 200 Watt, preferably of about 20 to 100 Watt, most preferably of about 45 Watt.

11. The composition according to any of claims 7 to 10, wherein the pulsed electromagnetic field has a magnetic field strength in the range of 100 to 150 µTesla.

12. Composition according to any of the preceding claims for use in medical treatment.

13. Composition according to any of the claims 1 to 11 for use in the treatment of type II diabetes mellitus.

14. Composition for use in treatment of type II diabetes mellitus according to claim 13, wherein the treatment comprises administering to a mammal, the composition comprising
a) at least 60 mg of the herbal extract per kg bodyweight of the mammal,
b) at least 120 mg of curcumin per kg bodyweight of the mammal; and
c) at least 10 mg quercetin per kg bodyweight of the mammal.
